# EUROPEAN PATENT SPECIFICATION

(11) **EP 1 915 951 B1**
(45) Date of publication and mention of the grant of the patent: **29.06.2011**
(21) Application number: 07254164.2
(22) Date of filing: 19.10.2007
(51) Int. Cl.: A61B 17/02, A61B 19/00

(54) **Knee distractor**
Kniegelenkdistraktor
Écarteur pour genou

(30) Priority: 26.10.2006 GB 0621341
(43) Date of publication of application: 30.04.2008
(73) Proprietor: Howmedica Osteonics Corp., Allendale, NJ 07401-1677 (US)
(72) Inventor: Vielpeau, Claude, 14200 Herouville St. Clair (FR); Mailhe, Didier, 34980 Murles (FR); Ghestem, Didier, 62580 Neuville Saint Vaast (FR); Rivat, Paul, 07130 St. Peray (FR); Raguet, Marc, 51000 Chalons en Champagne (FR); Stahl, Philippe, 59262 Sainghin en Melantois (FR); François, Jean-Marie, 67500 Marienthal (FR); Geais, Laurent, 94450 Limeil Brevannes (FR)
(74) Representative: Bridge-Butler, Alan James

(56) References cited:
- DE-A1-102005 049 851
- GB-A- 1 386 828
- US-A1- 2006 149 277

## Description

This invention relates to a knee distracter which can be used, for example, during the insertion of a prosthesis.

When prostheses are to be implanted, or for other operations involving the knee, it is often necessary to space the tibia away from the femur and, moreover, it is desirable to be able to act on the condyles of the femur so that they can be displaced individually and to varying degrees.

WO 2004/078047 shows a knee distracter which allows the above operations to be carried out but which employs flexible control means using a system of cables or a pneumatic arrangement.

Such control means are difficult to actuate in the operating theatre and are often inaccurate.

DE 10 2005 049 851 A1 shows a knee distracter with upper and lower elements with means for relative displacement to increase their distance from each other and shows displacement means having a scissors-type guide means which supports the appropriate elements in both the ventral-dorsal and the medial-lateral parallel positions. In the only embodiment actually shown the displacement means are activated by a lockable spring element which extends between the upper and lower elements for driving them apart. In this arrangement there is no means for controlling the action of the spring which merely springs open when it is released, pushing the element as far apart as possible In the circumstances. This lack of control could cause considerable damage to the patient's joint and, as there are no controls, the distance which the patient's bones are forced apart is entirely dependent upon the spring strength. Moreover, there is no provision for decreasing the distance between the elements so there is no way to control the removal of the distracter. There is further reference to this uncontrollable spring being replaced with an electromotive, electromagnetic, hydraulic or a pneumatic drive means. However, there is no disclosure of employing such alternative drive means to control the position of the upper and lower elements relative to one another to overcome the above described problems.

The present invention is intended to provide a knee distracter in which accurate and positive control of the parts can be achieved.

According to the present invention a knee distracter comprises a lower element, two upper elements located above the lower element, and operating means for moving each upper element independently to adjust its distance from the lower element, in which said operating means for each upper element comprises first and second crossed arms which are pivoted together at intermediate points in their lengths to provide a scissors action between them, an upper first end of a first arm being connected by a fixed pivot to an upper element and a second lower end of said first arm being connected by a sliding pivot to the lower element, a first lower end of the second arm being connected by a fixed pivot to the lower element and a second upper end of said second arm being connected by a sliding pivot to the upper element, and in which adjustment means are provided for adjusting the distance between each upper element and the lower element as required, characterised in that said adjustment means includes a screw jack for drawing the sliding pivot of the second arm connecting it to one element towards or away from the fixed pivot of the first arm connecting it to the same element to select any the distance between that upper element and the lower element as required.

The use of the pivoted crossed arms provides accurate control of the spaced between the upper and lower elements.

Each of the screw jacks may include a rotatable operating screw which is located substantially in the upper element and parallel with the upper surface thereof.

The rotatable operating screw can act on a threaded nut which is pivoted to the sliding pivot of the first end of the second arm.

Preferably, each upper element is provided with a pair of spaced apart first and second crossed arms, the axes and pivots of the first crossed arms being coaxial with the axes and pivots of the second crossed arms.

With this arrangement the said screw jacks can be located between a pair of crossed arms.

In a preferred construction each upper element comprises two parallel plates which locate between them the fixed pivot on the first arm and provide a slide to locate the sliding pivot on the second arm, and locate the screw jack.

With this construction the lower element may also comprise two parallel plates which locate between them the fixed pivot on the second arm and provide a slide to locate the sliding pivot on the first arm.

In all the above constructions the lower surface of the upper elements preferably lie in parallel engagement with the upper surface of the lower element when in a closed position.

The axis of rotation of the screw jacks may be arranged to extend in a posterior/anterior direction.

The invention can be performed in various ways and some embodiments will now be described by way of example and with reference to the accompanying drawings in which :
Figure 1 is an isometric view of the knee distracter according to the present invention from the anterior side with one of the upper elements in the raised position and the other upper element in its lowest position;
Figure 2 is a similar view to that shown in Figure 1 with one upper element in the raised position but with its top plate removed to reveal the mounting arrangements;

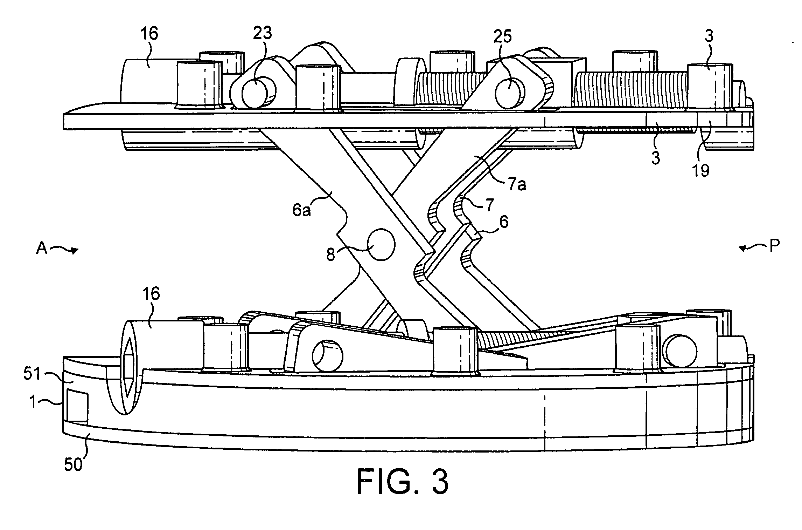

Figure 3 is a side elevation of the construction shown in Figures 1 and 2 from the lateral side with the medial upper element again in the raised position and the lateral upper element in its lowermost position and with the upper plates of both of the upper elements removed;
Figure 4 is a similar view to Figures 1 and 3 with the upper plates of the medial and lateral upper elements removed and with the upper plate of the lower element also removed;
Figure 5 is an isometric view of the lower surface of one of the upper elements from its anterior side;
Figure 6 is a similar view to Figure 5 with one of the screw jack screws in place;
Figure 7 is a similar view to Figure 6 but with part of the operating mechanism in the form of a threaded nut in place;
Figure 8 is a view similar to that shown in Figure 7 but with the crossed arms in place and located by the lower plate of the element;
Figure 9 is an isometric view from above and from the distal side of the lower plate of the lower element partly assembled with its two pairs of crossed arms, the crossed arms for the medial upper element being shown in the raised position and the crossed arms for the lateral upper element being in the lower position, the lateral screw jack is also shown;
Figure 10 is an isometric view of the upper and lower plates of the lower element taken from the posterior side; and,
Figure 11 is an isometric view from the posterior side of the two plates shown in Figure 10 but from beneath;

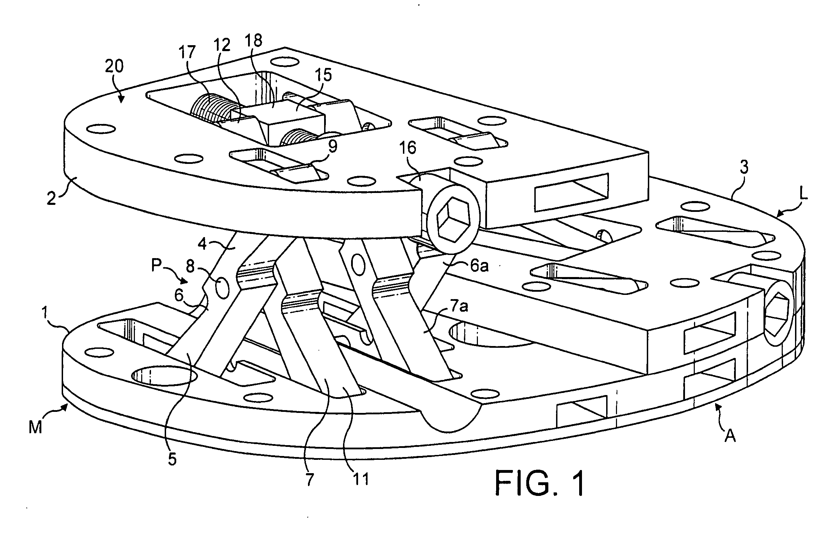

As shown in Figure 1 the assembled distracter comprises a lower element 1 and two upper elements 2 and 3. The anterior side of the device is indicated by reference numeral P, the medial side by reference numeral M and the lateral side by reference numeral L. The medial and lateral upper elements 2 and 3 are located above the lower element 1 and operating means 4 for moving each upper element independently to adjust its distance from the lower element 1 is provided. The operating means 4 for each upper element 2 or 3 comprises first and second crossed arms 6 and 7 respectively which are pivoted together at intermediate points 8 in their lengths to provide a scissors action between them. A first end 9 of the first arm 6 is connected by a fixed pivot 10 (not shown in Figure 1) to the upper element 2 and a second end 5 of this first arm 6 is connected by a sliding pivot (not shown in Figure 1) to the lower element 1. A first end 11 of the second arm 7 is connected by a fixed pivot (not shown in Figure 1) to the lower element 1 and a second end 12 of said second arm 7 being connected by sliding pivot (not shown in Figure 1) to the upper element 2. Means are provided for drawing the sliding pivot on one arm towards or away from the fixed pivot on the other arm to cause the distance between the upper and lower elements to be adjusted. In the construction being described this is achieved by means of a screw jack 15 which comprises a rotatable operating screw 16, the anterior end of which is provided with a head to received an operating allen key. The threaded part 17 of the screw 16 carries a threaded nut 18 which is pivoted to the sliding pivot on the end 12 of the second arm 7 so that rotation of the screw 16 causes the end 12 of the second arm 7 to approach the fixed pivot on the first end 9 of the other arm 6 or moves them apart thus raising and lowering the upper element in relation to the lower element.

In the embodiment described each of the upper elements 2 and 3 is provided with a pair of crossed arms, the second of the crossed arms as shown in Figure 1 being indicated by reference numerals 6a and 7a. The axes and pivots of the first crossed arms 6 and 7 are coaxial with the axes and pivots of the second crossed arms 6a, 7a.

The second arms 7 and 7a are arranged so that they are the closest arms to the screw jack 15, the arms 6 and 6a being outside them.

The rotatable operating screw 16 is located in the upper element 2 substantially parallel with the upper surface 20 thereof and the screw is located between the pair of crossed arms 6, 7 and 6a, 7a.

The same mechanism is also provided in the lateral upper plate 3 and on the lateral side of the lower element 1.

It will be appreciated that each of the upper elements 2 or 3 can be operated independently of the other to provide the desired spacing when separating the bones of the knee.

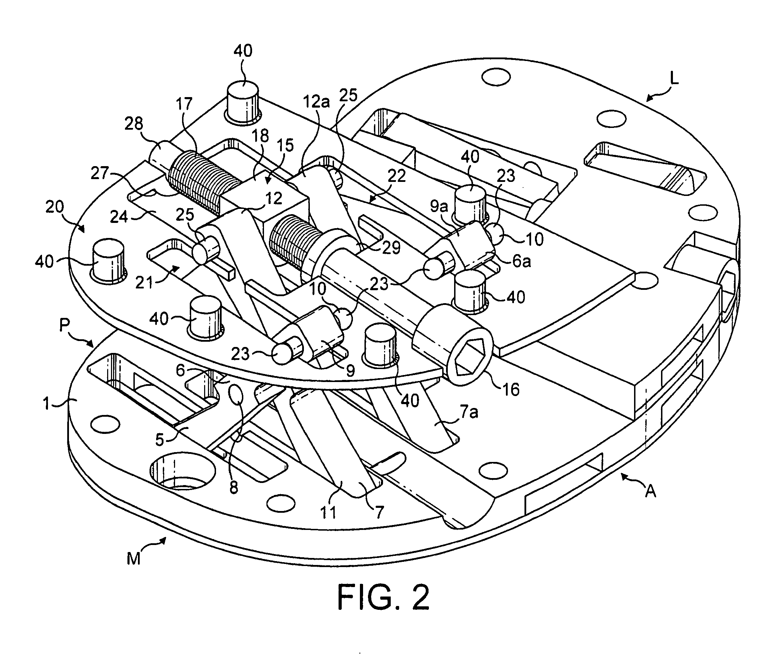

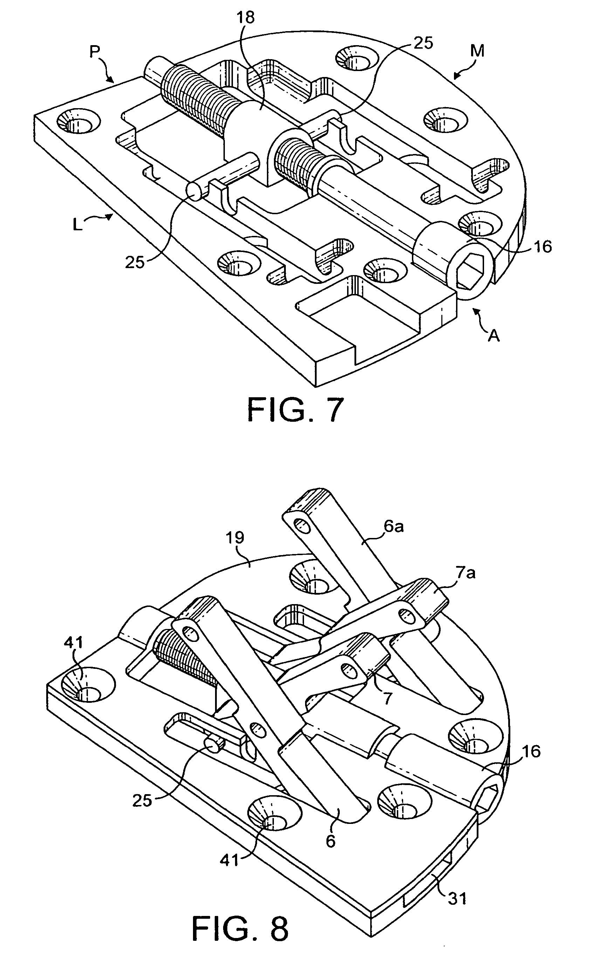

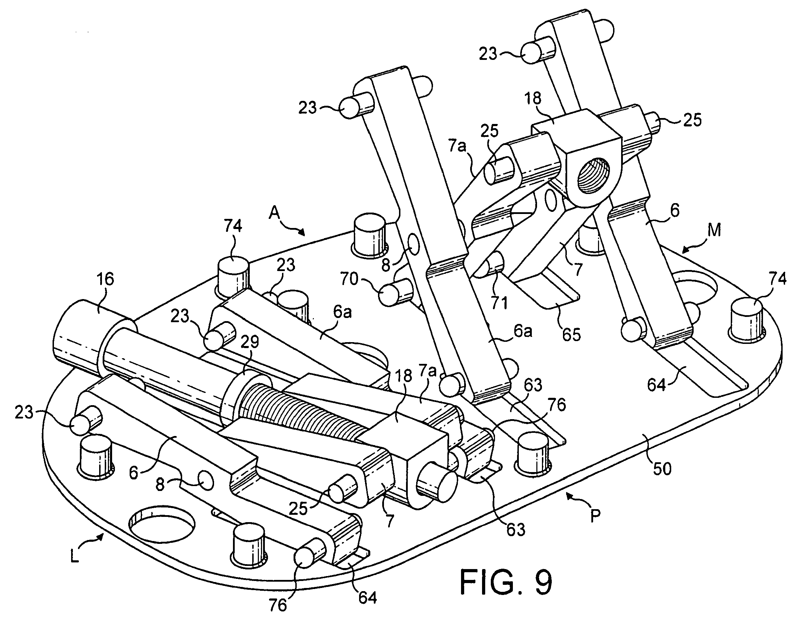

Each upper element 2 or 3 is formed from upper and lower plates which are shown in Figures 2, 5, 6 and 7 and when assembled in Figure 8 and the lower element 1 is also made up from upper and lower plates which are shown in Figures 9, 10 and 11.

Each of the lower plates 19 on the upper elements 2 and 3 is substantially flat and has two openings 21 and 22 to respectively receive the ends 9 and 9a of the first crossed arms 6 and 6a.

The ends 9 and 9a and the arms 6 and 6a carry trunnions 23 which, when the apparatus is assembled, rest against the upper surface of the lower plate 19 which retains them in place further. An opening 24 is provided in the plate to accommodate the ends 12 and 12a of the arms 7 and 7a together with the nut 18 of the screw jack 15. Each of the ends 12, 12a is provided with trunnions 25 which extend on their outer ends over part of the plate 19 on each side, the other ends of the trunnions being housed in the nut 18 to provide a pivotal joint. The posterior side of the plate 19 is provided with a downwardly extending projection 27 to form a split bearing to accommodate the end 28 of the screw 16. Screw 16 also has a location flange 29 which bears against one side of the opening 24 and which co-operates with the head of the screw to locate it axially in place in the plate 19.

The construction described above is the same for both upper elements.

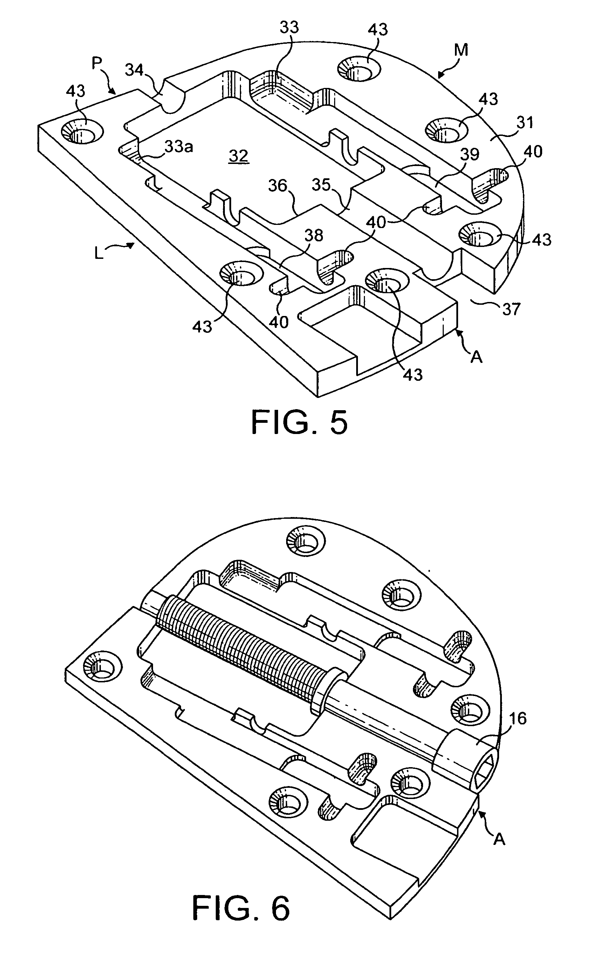

The various parts are held in place on each of the upper elements by an upper plate 31, the lower surface of which is shown Figures 5, 6 and 7. Each upper plate 31 has a central opening 32 to accommodate the upper ends 12 and 12a of the crossed arms 7, 7a and the screw jack mechanism 15. When the upper plate 31 is located in place the trunnions 25 on the ends 12a of the arms are located in open sided grooves 33, 33a respectively. Thus the ends of the arms can move in anterior/posterior directions as a sliding pivot in the grooves but are restrained from upwards and downwards movement.

The nut 18 which is located between the two arms is prevented from rotating on the screw 16 and the end 28 of the screw is located in a bearing recess 34. The anterior end of the screw is located in a further recess 35 and is free to rotate therein. The end 36 of the opening 32 engages the flange 29 on the screw 16 and the screw head is located in a recess 37 in the anterior edge of the plate.

Two further openings 38 and 39 are provided through which the ends 9 and 9a of the arms 6, 6a can project and their trunnions 23 are located in bearing recesses 4 which, when the plates are assembled, act to provide fixed pivots for the ends 9 of the arms 6.

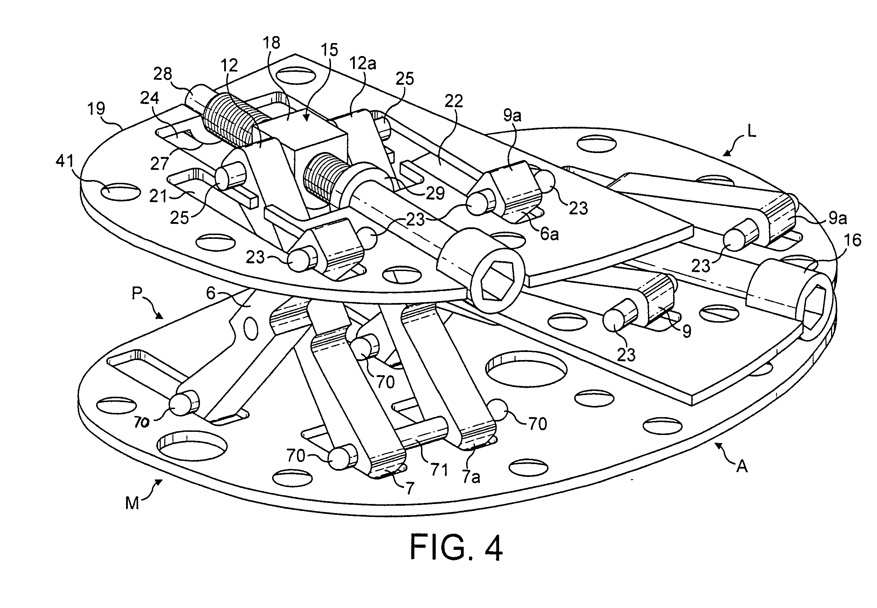

Upper plate 34 is connected to lower plate 19 by set screws indicated by reference numeral 40 in Figure 2. These set screws 40 are not shown in Figure 4 but the holes to receive them are indicated by reference numeral 41. The set screws 40 are screwed into threaded openings 43 which are shown in Figures 5, 6 and 7.

Figure 6 is a view similar to Figure 5 showing the set screw 16 in position and Figure 7 shows the screw 16 together with its nut 18 located in the opening 24. The trunnions 25 at the ends 12, 12a of the arms 7, 7a are shown as rods which extend through them with protruding ends, the arms themselves either being fixed and the trunnions rotating in the nut 18 and against the plate 31 or being held rigidly in the nut 18 and locating against the ends of the arms 12, 12a.

Figure 8 shows the upper plate 34 connected to the lower plate with the crossed arms 6, 6a and 7, 7a assembled in position together with the screw 16. In this Figure the pins which provide the pivot 8 and trunnions which connect to the lower plate 1 (to be described hereunder) have not yet been inserted and the screws 40 have not been entered into the screw holes 41 or screw threaded openings 43.

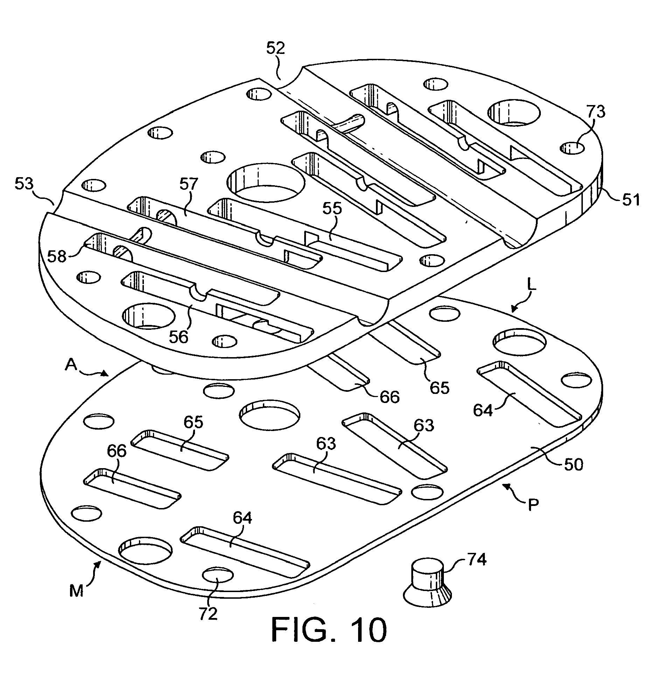

The construction of the lower element 1 includes a substantially flat lower plate 50 (as shown in Figure 10) and a co-operating upper plate 51.

The upper plate 51 has troughs 52 and 53 to accommodate the lower surfaces of the screw 16 when the upper elements 2 and 3 are in their closed and lowest position.

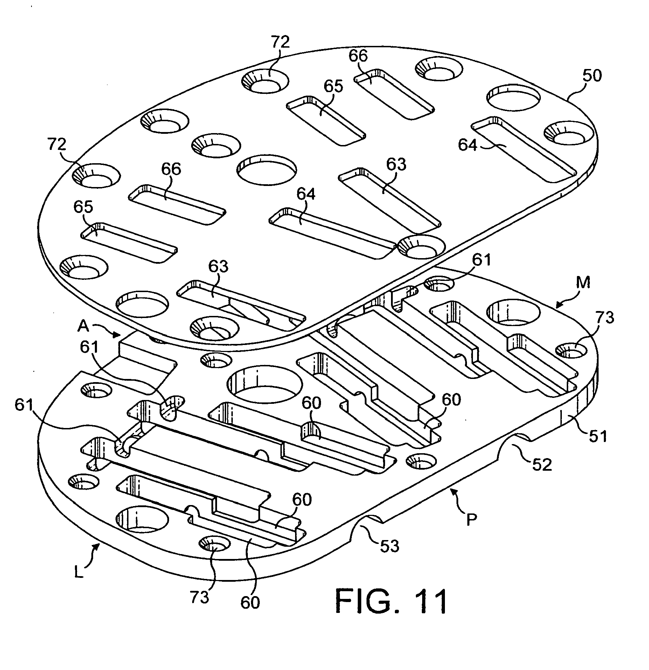

The upper plate 51 also has spaced parallel openings 55 and 56 to receive and accommodate the lower ends 5 of the crossed arms 6, 6a and there are further parallel openings 57 and 58 to receive and accommodate the lower ends 11 of the crossed arms 7, 7a. The lower surface of the upper plate 51, as shown in Figure 11, is provided with recesses 60 to accommodate trunnions on the crossed arms (to be described). Trunnion location sockets 61 are also provided.

The arrangement is repeated on the medial side of the plate to accommodate the operating apparatus for the medial upper element.

The lower plate 50 is provided with openings 63, 64 to accommodate the lower ends of the crossed arms 7, 7a, on each side, and further parallel openings 65, 66 to accommodate the lower ends of the crossed arms 6 on each side.

As is clear from Figure 4 the lower end 5 of the crossed arm 6 and lower ends 11 of the arms 7 carry projecting trunnions 70 and the trunnions 70 extending between the arms 7, 7a are provided as a single pin 71.

The trunnions 70 are located appropriately in the recesses 60 and location sockets 61 to provide fixed pivots in the sockets 61 and sliding pivots in the recesses 60 in a similar manner to the construction of the upper elements.

Screw receiving holes 72 are provided in the lower plate 50 and screw threaded holes 73 in the upper plate 51 to receive screws 74, as shown in Figure 10, to hold the two plates together.

Figure 9 shows the various crossed arms and one of the screw jacks in position and resting on the lower plate 50. The lateral crossed arms are shown in the lowered position and the medial crossed arms in a raised position. In this Figure the upper elements and the upper plate 51 of the lower element are not shown. The invention provides a construction which enables the two upper elements to be operated independently utilising a reliable and strong operating mechanism which can be easily operated in the operating theatre and does not require any further apparatus apart from an allen key to operate the screw jacks.

Due to the construction of the device according to the present invention knee balance stability in flexion and extension can be accessed with the patella in place.

## Claims

1. A knee distracter comprising a lower element (1), two upper elements (2)(3) located above the lower element (1), and operating means (4) for moving each upper element (2)(3) independently to adjust its distance from the lower element (1), in which said operating means (4) for each upper element (2)(3) comprises first and second crossed arms (6)(7) which are pivoted together at intermediate points (8) in their lengths to provide a scissors action between them, an upper first end (9) of a first arm (6) being connected by a fixed pivot (10) to an upper element (2)(3) and a second lower end (5) of said first arm (6) being connected by a sliding pivot to the lower element (1), a lower first end (11) of the second arm (7) being connected by a fixed pivot to the lower element (1) and a second upper end (12) of said second arm (7) being connected by a sliding pivot to the upper element (2)(3), and in which adjustment means (15) are provided for adjusting the distance between each upper element (2)(3) and the lower element (1) as required, **characterised in that** said adjustment means (15) includes a screw jack (15) for drawing the sliding pivot of the second arm (7) connecting it to one element (2)(3) towards or away from the fixed pivot (10) of the first arm (6) connecting it to the same element (2)(3) to select any distance between that upper element (2)(3) and the lower element (1) as required.

2. A knee distracter as claimed in claim 1 **characterised in that** each of the screw jacks (15) includes a rotatable operating screw (16) which is located substantially in the upper element (2)(3) and parallel with the upper surface thereof.

3. A knee distracter as claimed in claim 2 **characterised in that** the rotatable operating screw (16) acts on a threaded nut (18) which is pivoted to the sliding pivot of the first end (12) of the second arm (7).

4. A knee distracter as claimed in any one of the preceding claims **characterised in that** each upper element (2)(3) is provided with a pair of spaced apart first and second crossed arms (6)(7), the axes and pivots of the first crossed arms (6) being coaxial with the axes and pivots of the second crossed arms (7).

5. A knee distracter as claimed in claim 4 **characterised in that** said screw jacks (15) are located between a pair of crossed arms (6)(7).

6. A knee distracter as claimed in any one of the preceding claims **characterised in that** each upper element (2)(3) comprises two parallel plates which locate between them the fixed pivot (10) on the first arm (6) and provide a slide to locate the sliding pivot on the second arm (7), and locate the screw jack (15).

7. A knee distracter as claimed in claim 6 **characterised in that** the lower element (1) also comprises two parallel plates which locate between them the fixed pivot on the second arm (7) and provide a slide to locate the sliding pivot on the first arm (6).

8. A knee distracter as claimed in claim 6 or claim 7 **characterised in that** the lower surface of the upper elements (2)(3) lie in parallel engagement with the upper surface of the lower element (1) when in a closed position.

9. A knee distracter as claimed in any one of the preceding claims **characterised in that** axes of rotation of the screw jacks (15) are arranged to extend in a posterior/anterior direction.

## Patentansprüche

1. Knie-Distraktor, umfassend ein unteres Element (1), zwei oberhalb von dem unteren Element positionierte obere Elemente (2)(3), sowie ein Betätigungsmittel (4), um jedes obere Element (2)(3) unabhängig zu bewegen, um ihren Abstand von dem unteren Element (1) zu verstellen, bei dem das Betätigungsmittel (4) für jedes obere Element (2)(3) einen gekreuzten ersten und zweiten Arm (6)(7) umfasst, die an Zwischenpunkten (8) ihrer Länge aneinander angelenkt sind, um für eine Scherenbewegung zwischen ihnen zu sorgen, wobei ein oberes erstes Ende (9) eines ersten Arms (6) durch einen festen Drehzapfen (10) mit einem oberen Element (2)(3) verbunden ist und ein zweites unteres Ende (5) des ersten Arms (6) durch einen gleitenden Drehzapfen mit dem unteren Element (1) verbunden ist, wobei ein unteres erstes Ende (11) des zweiten Arms (7) durch einen festen Drehzapfen mit dem unteren Element (1) verbunden ist und ein zweites oberes Ende (12) des zweiten Arms (7) durch einen gleitenden Drehzapfen mit dem oberen Element (2)(3) verbunden ist, und bei dem Verstellmittel vorgesehen sind, um den Abstand zwischen jedem oberen Element (2)(3) und dem unteren Element (1) wie gewünscht zu verstellen, **dadurch gekennzeichnet, dass** das Verstellmittel (15) eine Schraubenwinde (15) einschließt, um den gleitenden Drehzapfen des zweiten Arms (7), der ihn mit einem Element (2)(3) verbindet, zum festen Drehzapfen (10) des ersten Arms (6), der ihn mit demselben Element (2)(3) verbindet, hin oder von diesem weg zu ziehen, um einen beliebigen Abstand, wie gewünscht, zwischen diesem oberen Element (2)(3) und dem unteren Element (1) auszuwählen.

2. Knie-Distraktor nach Anspruch 1, **dadurch gekennzeichnet, dass** jede von den Schraubenwinden (15) eine drehbare Betätigungsschraube (16) einschließt, die im Wesentlichen im oberen Element (2)(3) positioniert und zu dessen Oberseite parallel ist.

3. Knie-Distraktor nach Anspruch 2, **dadurch gekennzeichnet, dass** die drehbare Betätigungsschraube (16) auf eine Gewindemutter (18) wirkt, die an dem gleitenden Drehzapfen des ersten Endes (12) des zweiten Arms (7) angelenkt ist.

4. Knie-Distraktor nach einem der vorangehenden Ansprüche, **dadurch gekennzeichnet, dass** jedes obere Element (2)(3) mit einem Paar von im Abstand voneinander angeordneten gekreuzten ersten und zweiten Armen (6)(7) versehen ist, wobei die Achsen und Drehzapfen der ersten gekreuzten Arme (6) koaxial zu den Achsen und Drehzapfen der zweiten gekreuzten Arme (7) sind.

5. Knie-Distraktor nach Anspruch 4, **dadurch gekennzeichnet, dass** die Schraubenwinden (15) zwischen einem Paar von gekreuzten Armen (6)(7) positioniert sind.

6. Knie-Distraktor nach einem der vorangehenden Ansprüche, **dadurch gekennzeichnet, dass** jedes obere Element (2)(3) zwei parallele Platten umfasst, die zwischen sich den festen Drehzapfen (10) auf dem ersten Arm (6) positionieren und für eine Gleitführung sorgen, um den gleitenden Drehzapfen auf dem zweiten Arm (7) zu positionieren, und die Schraubenwinde (15) positionieren.

7. Knie-Distraktor nach Anspruch 6, **dadurch gekennzeichnet, dass** das untere Element (1) ebenfalls zwei parallele Platten umfasst, die zwischen sich den festen Drehzapfen auf dem zweiten Arm (7) positionieren und für eine Gleitführung sorgen, um den gleitenden Drehzapfen auf dem ersten Arm (6) zu positionieren.

8. Knie-Distraktor nach Anspruch 6 oder Anspruch 7, **dadurch gekennzeichnet, dass** die Unterseite der oberen Elemente (2)(3) in parallelem Eingriff mit der Oberseite des unteren Elements (1) liegt, wenn er sich in einer geschlossenen Stellung befindet.

9. Knie-Distraktor nach einem der vorangehenden Ansprüche, **dadurch gekennzeichnet, dass** Drehachsen der Schraubenwinden (15) angeordnet sind, um sich in einer posterior/anterioren Richtung zu erstrecken.

## Revendications

1. Ecarteur pour genou, comprenant un élément inférieur (1), deux éléments supérieurs (2, 3) situés au-dessus de l'élément inférieur (1) et un moyen d'actionnement (4) pour déplacer indépendamment chaque élément supérieur (2, 3) afin de régler sa distance par rapport à l'élément inférieur (1), dans lequel ledit moyen d'actionnement (4) pour chaque élément supérieur (2, 3) comporte une première et une seconde branches en croix (6, 7) qui sont amenées à pivoter l'une par rapport à l'autre pour créer une action de ciseaux entre elles, une première extrémité supérieure (9) d'une première branche (6) étant accouplée par un pivot fixe (10) avec un élément supérieur (2, 3) et une seconde extrémité inférieure (5) de ladite première branche (6) étant accouplée par un pivot coulissant avec l'élément inférieur (1), une première extrémité inférieure (11) de la seconde branche (7) étant accouplée par un pivot fixe avec l'élément inférieur (1) et une seconde extrémité supérieure (12) de ladite seconde branche (7) étant accouplée par un pivot coulissant avec l'élément supérieur (2, 3), et dans lequel un moyen de réglage (15) est prévu pour régler la distance requise entre chaque élément supérieur (2, 3) et l'élément inférieur (1), **caractérisé en ce que** ledit moyen de réglage (15) comporte un vérin (15) à vis pour tirer le pivot coulissant de la seconde branche (7) accouplant celle-ci avec un élément (2, 3) dans le but de le rapprocher ou de l'éloigner du pivot fixe (10) de la première branche (6) l'accouplant au même élément (2, 3) afin de sélectionner n'importe quelle distance requise entre cet élément supérieur (2, 3) et l'élément inférieur (1).

2. Ecarteur pour genou selon la revendication 1, **caractérisé en ce que** chacun des vérins (15) à vis comporte une vis rotative d'actionnement (16) située sensiblement dans l'élément supérieur (2, 3) et parallèle à la surface supérieure de celui-ci.

3. Ecarteur pour genou selon la revendication 2, **caractérisé en ce que** la vis rotative d'actionnement (16) agit sur un écrou fileté (18) amené à pivoter par rapport au pivot coulissant de la première extrémité (12) de la seconde branche (7).

4. Ecarteur pour genou selon l'une quelconque des revendications précédentes, **caractérisé en ce que** chaque élément supérieur (2, 3) est pourvu d'une paire de première et seconde branches (6, 7) en croix espacées l'une de l'autre, les axes et pivots des premières branches (6) en croix étant coaxiaux aux axes et pivots des secondes branches (7) en croix.

5. Ecarteur pour genou selon la revendication 4, **caractérisé en ce que** lesdits vérins (15) à vis sont situés entre les branches d'une paire de branches (6, 7) en croix.

6. Ecarteur pour genou selon l'une quelconque des revendications précédentes, **caractérisé en ce que** chaque élément supérieur (2, 3) comporte deux plaques parallèles qui déterminent entre elles la position du pivot fixe (10) sur la première branche (6) et permettent un coulissement pour déterminer la position du pivot coulissant sur la seconde branche (7) et la position du vérin (15) à vis.

7. Ecarteur pour genou selon la revendication 6, **caractérisé en ce que** l'élément inférieur (1) comporte aussi deux plaques parallèles qui déterminent entre elles la position du pivot fixe sur la seconde branche (7) et permettent un coulissement pour déterminer la position du pivot coulissant sur la première branche (6).

8. Ecarteur pour genou selon la revendication 6 ou la revendication 7, **caractérisé en ce que**, en position fermée, la surface inférieure des éléments supérieurs (2, 3) se trouve parallèlement au contact de la surface supérieure de l'élément inférieur (1).

9. Ecarteur pour genou selon l'une quelconque des revendications précédentes, **caractérisé en ce que** les axes de rotation des vérins (15) à vis sont conçus pour s'étendre dans une direction postéro-antérieure.
